# EUROPEAN PATENT APPLICATION

(11) **EP 4 704 107 A1**
(43) Date of publication of application: **04.03.2026**
(21) Application number: 24197269.4
(22) Date of filing: 29.08.2024
(51) Int. Cl.: G16H 40/20

(54) **A METHOD AND SYSTEM FOR MONITORING AN ASSET IN REAL-TIME**

(71) Applicant: Controlant hf., 201 Kopavogur (IS)
(72) Inventor: ARNASON, Gudmundur Arni, 201 Kopavogur (IS); BRYNJULFSSON, Erlingur, 201 Kopavogur (IS); TIPTON, Lee Roy, 201 Kopavogur (IS)
(74) Representative: Inspicos P/S

(57) **Abstract**

This invention relates to a method and a system for real-time monitoring of an asset using a logger device associated with the asset, where the logger device comprises:
• a sensor for measuring an environment related parameter of the asset,
• a memory for storing the measured environment related parameter,
• a transmitter for transmitting via a communication network the stored measured environment related parameter to an external computer device with a pre-defined time interval,
• a display, and
• a processor for operating the sensor, the memory, the communication module and the display, wherein the method comprises:
• identifying, by the processor, if there is a communication gap between the logger device and the external computer device, where in case a communication gap is identified,
converting, by the processor, received and stored environment related parameters over a pre-defined time interval starting from the time point where the communication gap is identified into a Quick Response (QR) and present the QR code on the display.

## Description

### FIELD OF THE INVENTION

The present invention relates to a method and a system of real-time monitoring of an asset using a logger device associated with the asset.

### BACKGROUND OF THE INVENTION

Logger devices are electronic monitoring devices used for monitoring environment related parameters of assets such as pharmaceutical products, where a common environment related parameter is the temperature of the asset that is being monitored.

The logger devices may be used as an example for real-time monitoring where it is associated with the packaging containing the asset, e.g. on an outer side or the inner side, where the logger device may in real-time transmit the temperature of the asset together with the location to an external computer device. Thus, issues such as the temperature of the asset being too high or too low may be identified before these issues escalate which allows for proactive actions to prevent the assets from being damaged. Also, if the asset is a pharmaceutical product, upon arrival at a destination location, which may as an example be a hospital, it may not be released and administered to a patient unless reliable data is available showing that e.g. the temperature during the transport was within a given range.

Another use case is where such logger devices are used for on-site monitoring for pharmaceutical products that may be kept at an isolated cooled area such as a freezer at a pre-fixed temperature range, e.g. below -20°C. Before administering a patient with a pharmaceutical product that has been stored for days or weeks, the pharmaceutical products must be released meaning that it must be possible to show that the storing conditions fulfilled storage requirement throughout this storage time.

In both cases, the logger devices typically rely on a use cellular coverage to be able to transmit the stored environmental related data to the external computer device, which may e.g. be every hour.

One of the challenges today is when a data gap occurs that might arise from a lack of cellular coverage. As an example, this might happen when the logger device tries to transmit the environmental related data such as the stored temperature data in the last one hour of the transport but is not able to do so, due to a lack of cellular coverage.

In case of the above-mentioned scenarios, if the scenario occurs that there is a data gap the last hours or last days before administering a drug to a patient, the drug may not be released and thus may not be administered to the patient. This can obviously result in a risk fort the patient.

### SUMMARY OF THE INVENTION

It is an object of the invention to provide a method and a system that enables collecting data during a communication gap, e.g. as to allow pharmaceutical products to be released immediately.

In general, the invention preferably seeks to mitigate, alleviate or eliminate one or more of the above-mentioned disadvantages of the prior art singly or in any combination. In particular, it may be seen as an object of embodiments of the present invention to provide a method and a system that solves the above-mentioned problems, or other problems.

In a first aspect, a method is provided of real-time monitoring of an asset such as a pharmaceutical product using a logger device associated with the asset, where the logger device comprises:
- a sensor for measuring an environment related parameter of the asset,
- a memory for storing the measured environment related parameter,
- a transmitter for transmitting via a communication network the stored measured environment related parameter to an external computer device with a pre-defined time interval,
- a display, and
- a processor for operating the sensor, the memory, the communication module and the display,
wherein the method comprises:
- identifying, by the processor, if there is a communication gap between the logger device and the external computer device, where in case a communication gap is identified,
- converting, by the processor, received and stored environment related parameters over a pre-defined time interval starting from the time point where the communication gap is identified into a Quick Response (QR) and present the QR code on the display.

The data gap may therefore be overcome by storing the data in the QR code, that may later on be scanned by a scanning device, e.g. a mobile phone or any type of portable computer device. This will as an example be crucial when a patient is to be administered with a pharmaceutical product because the product may immediately be released and administered to the patient based on the data converted into the QR code, assuming the data are fulfilling pre-defined storing conditions.

As an example, the data stored in the QR code may be retrieved based on the scanning, where the scanning may trigger a service request that is sent to a server individuated by the QR code. Using the same service request object's state data, measurement data contained in the QR code may then be transmitted to the server and in that way the data may be accessed and processed to e.g. determine if the pharmaceutical product may be released or not. A common criterion is to be able to show that the temperature of the asset was within a certain temperature interval or e.g. below a certain temperature range.

In an embodiment, the logger device is uniquely identified via a unique identification (ID), where the QR code further contains the unique ID. This unique ID may as an example comprise a unique serial number of the logger device and in that way from which logger device the transmitted data originate from.

In an embodiment, the step of converting comprises:
- converting received and stored environment related parameters over discrete time interval into a QR code, where each discrete time interval is shorter than the pre-defined time interval, and
- gradually updating the QR code for each of said discrete time interval with increased number of stored environment related parameters from previous time interval until the expiry of the pre-defined time interval.

As an example, the pre-defined time-period may be 12 hours, whereas the discrete time interval may be 1 hour. In that way, the converted environment related parameters QR code is more frequently converted into the QR code meaning that when asset is to be used, e.g. the drug administered to the patient before the 12 hours period, the data up to that time-point may be retrieved.

In an embodiment, the step of converting comprises converting the received and stored environment related parameters accumulated over the pre-defined time interval into a single QR code. This may result in power saving of the energy source.

In an embodiment, the asset together with the logger device is stationary during the real-time monitoring and where the asset is placed in a temperature-controlled area. This would be the scenario where the logger device is used for on-site monitoring for sensitive assets such as pharmaceutical products that may be kept at an isolated cooled area such as a freezer at a pre-fixed temperature range, e.g. below -20°C or within a given temperature window. Before administering a patient with a pharmaceutical product that has been stored for days or weeks, the pharmaceutical products must be released meaning that it must be possible to show that the storing conditions fulfilled storage requirement. In case of said communication gap, the releasing process may take place immediately and the patient may be administered the pharmaceutical product.

In an embodiment, the asset together with the logger device is transported from a begin location to a destination location during the real-time monitoring, where the method further comprises embedding position data to the QR code and transmitting the position data of the logger device together with the stored measured environment related parameter.

In an embodiment, the method further comprises compressing the received and stored environment related parameters prior to converting it into the QR code. In that way the amount of environment related parameters may be converted into the QR code.

In an embodiment, the pre-defined time interval is selected such that the resolution of the QR code when displayed on the display is above a pre-defined resolution range. In that way, a data quality and resolution of the data converted into the QR code is ensured. As an example, in pharmaceutical industries there is a requirement that there is minimal data resolution of e.g. measured temperature data, e.g. 1 hour frequency. In an embodiment, when the resolution of the QR code reaches the pre-defined resolution range a subsequent QR code is generated and where the pre-defined time for the subsequent QR code is selected such that the resolution of the subsequent QR code when displayed on the display is above the pre-defined resolution range. This may then be repeated where multiple of QR codes may be created. In that way, data gap for the last hours or days before e.g. administering a patient with a pharmaceutical product, the pharmaceutical product may be released and given to the patient, simply by scanning the QR code(s), where the "missing" data due to e.g. lack of cellular connection, may immediately be retrieved.

This may also apply for other sensitive assets such as, but not limited to, food products and beverages.

In another embodiment, when the resolution of the QR code reaches the pre-defined resolution range the resolution range is reduced. This may as an example mean that registering every two- or three-hours environment related parameter instead of e.g. every 1 hour. This may be acceptable in some scenarios, e.g. when the asset is not very sensitive like a pharmaceutical product.

The environmental related parameter may be selected from, but is not limited to, one or more of:
- the temperature of the asset or around the asset,
- the ambience pressure,
- the vibration or acceleration of the asset,
- the light intensity around the asset.

In an embodiment, the method further comprises defining reference value or reference range of the environment related parameter indicating optimal transport conditions for the asset during the transport. This may as an example include a single reference range, or one or more reference windows. Sensitive asset such as a pharmaceutical product may be very sensitive for temperature, where e.g. an optimal storage range may be between 0°C and 2°C, or below 0°C, where if the temperature is outside this range a temperature excursion occurs. It is common that the temperature may be outside such defined ranges for a given time-period, sometimes referred to a as stability budget. In an embodiment, if an excursion occurs from the defined reference value or reference range the frequency of measured environment related parameter is increased. This may be the case that if the measured temperature as an example is either within said range or below said single reference value, there is no need to measure and stored the data in the QR code, or at least with less frequency, e.g. every 5 hours, 10 hours etc., whereas in case the temperature (or any other environmental related parameter is outside these ranges), it may be highly relevant to increase the measurement frequency, e.g. every 10 minutes, 30 minutes, to allow carefully monitoring the development of the measured temperature (environmental related parameter). In addition to the examples above, the temperature profile may be such that the measured temperature (or any other environment related parameter) may not be above and/or below certain values, e.g. not be above 30°C or below -30°C. It may therefore be highly critical to increase the measurement frequency if the measured environment related parameter is outside said temperature window or value.

In an embodiment, the method further comprises utilizing the stored environment related parameter as input in calculating one or more of the asset related parameters and converting the one or more of the asset related parameters into the QR code. The asset related parameters may as an example indicate if an excursion occurred, how long it lasted, average measured temperature related parameter etc., just to give a high-level overview.

In an embodiment, the method further comprises, upon an external action and prior to the expiry of the pre-defined time interval, a QR code is generated based on the stored data for a time-period starting from the time point where the communication gap is identified until the external action is received.

In a second aspect of the invention, a system is provided for real-time monitoring of an asset using a logger device associated with the asset, where the logger device comprises:
- a sensor for measuring an environment related parameter of the asset,
- a memory for storing the measured environment related parameter,
- a transmitter for transmitting via a communication network the stored measured environment related parameter to an external computer device with a pre-defined time interval,
- a display, and
- a processor for operating the sensor, the memory, the communication module and the display,
wherein the processor is configured to identify if there is a communication gap between the logger device and the external computer device, where in case a communication gap is identified, and convert the received and stored environment related parameters over a pre-defined time interval starting from the time point where the communication gap is identified into a Quick Response (QR) and present the QR code on the display.

The display may in one embodiment comprise an ink-display. In that way, an energy favourable solution is provided to display the QR on the display because it only requires processing power when the displayed image is updated or changed.

In an embodiment, the processor is further configured to compress the received and stored environment related parameters prior to converting it into the QR code.

The sensor may be selected from one or more of:
- temperature sensor for measuring the temperature of the asset or around the asset,
- a barometer for measuring the ambience pressure,
- an accelerometer for measuring the vibration or acceleration of the asset,
- a light sensor for measuring the light intensity around the asset.

In general, the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which:
Figure 1 depicts a flowchart of a method according to the present invention of real-time monitoring of an asset using a logger device associated with the asset while the asset is transported from an origin location to a destination location,
Figures 2 to 6 depict one use case scenario of a system present invention, which is a so-called on-site monitoring of pharmaceutical products, and
Figure 7 depicts a scenario where the asset together with the logger device is transported by a vehicle 610 from a begin location to a destination location during the real-time monitoring.

### DESCRIPTION OF EMBOIDMENTS

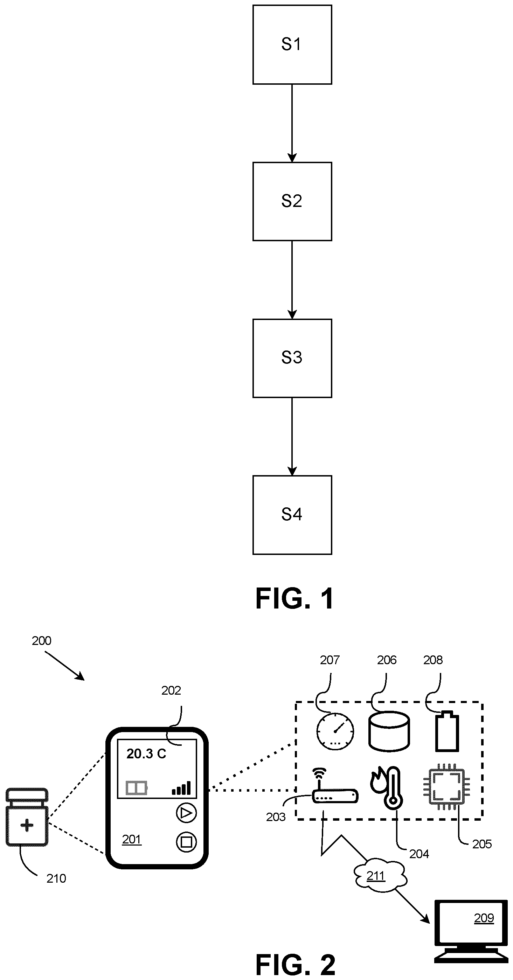

Figure 1 shows a flowchart of a method of real-time monitoring of an asset using a logger device associated with the asset while the asset is transported from an origin location to a destination location.

The asset may comprise a sensitive product such as a pharmaceutical product, and the environment related parameter is selected from, but is not limited to, the temperature of the asset or around the asset, the ambience pressure, the vibration or acceleration of the asset, the light intensity around the asset.

In a first step (S 1) 101, an identification processed identifies if there is a communication gap between the logger device and the external computer device that can be any type of a cloud platform system.

In a second step (S2) 102, in case a communication gap is identified, the received and
stored environment related parameters over a pre-defined time interval starting from the time point where the communication gap is identified are converted into a Quick Response (QR), where the QR code is presented on the display, which may comprise an e-ink display to safe energy.

The pre-defined time interval may in one embodiment be selected such that the resolution of the QR code when displayed on the display is above a pre-defined resolution range. If as an example the pre-defined time interval is 24 hours, and this time-period is coming to expiry, another QR code is generated and where the pre-defined time for the subsequent QR code is selected such that the resolution of the subsequent QR code when displayed on the display is above the pre-defined resolution range, in this case 24 hours.

In another alternative embodiment, when the resolution of the QR code reaches the pre-defined resolution range the resolution range is reduced. This may as an example be the case where every second, every third, etc. environmental related parameter is stored in that way the data resolution is reduce.

In a third step (S3) 103, the received and stored environment related parameters are compressed prior to converting the data into the QR code. In that way, the amount of data may obviously be increased.

In a fourth step (S4) 104, a reference value or reference range is defined for the environment related parameter indicating optimal storage conditions for the asset. This may as an example be reference window or several reference widows, or a single reference value. If an excursion occurs from the defined reference value or reference range the frequency of measured environment related parameter is increased. As an example, if the reference range is temperature t1 and temperature t2, where t2>t1, the temperature measurement while the measured temperature is within the range between t1 and t2, there may be no need to embed the measured temperature at all in the QR code, or with a low frequency, e.g. every 5 hours, whereas if the temperature is outside this range, the frequence of embedding this data is higher, or corresponding to the measurement frequency which may e.g. be every one hour.

Figure 2 shows a system 200 according to the present invention system of real-time monitoring of an asset 210 using a logger device 201 associated with the asset. This may as an example include attaching the logger 201 device to the packaging material carrying the asset or placing it into the packaging material containing the asset.

The logger device 201 comprises a sensor such as a temperature sensor 204 for measuring an environment related parameter of the asset, a memory 206 for storing the measured environment related parameter such as the measured temperature values, a transmitter 203 for transmitting via a communication network 211 the stored measured environment related parameter to an external computer device 209 with a pre-defined time interval, a display 202, a power source 208, and a processor 205 for operating the sensor, the memory, the communication module and the display.

The processor 205 is configured to identify if there is a communication gap between the logger device 201 and the external computer device 209, where in case a communication gap is identified, and convert the received and stored environment related parameters over a pre-defined time interval measured by a clock or similar means 207 starting from the time point where the communication gap is identified into a Quick Response (QR) and present the QR code on the display.

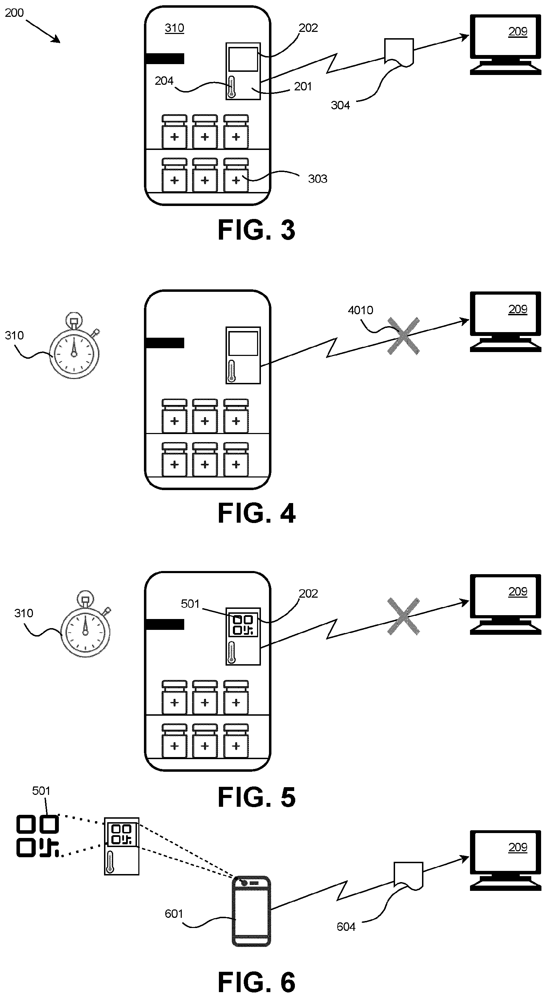

Figures 3 to 6 depict one use case scenario of the system 200 discussed in relation to figure 2, which is a so-called on-site monitoring of pharmaceutical products, where such pharmaceutical products may be stored for days, weeks, months or even more before they are administered to a patient. When storing such a sensitive product, it is of outmost importance that the temperature has been within a pre-defined range or e.g. below/above a temperature value during this storage time. Having this information available the product may immediately be release and be administered to a patient if the storage conditions .

Figure 3 shows a cooling equipment which may be refrigerator or a freezer 310 for preserving the pharmaceutical product 303, where the temperature is measured by a temperature sensor 204 comprised in a logger device 201 and saved in a storage medium, where the stored temperature data 204 is regularly transmitted, e.g. every 1 hour, to an external control computer 205.

Figure 4 depicts the scenario where the processor 205 identifies a communication gap 401 between the logger device 201 and the external computer device 209.

From the moment where the communication gap is identified the received and stored environment related parameters over a pre-defined time interval 310 starting from the time point where the communication gap is identified is converted into a Quick Response (QR) 401, where the QR code is subsequently presented on the display 202 as shown in figure 5. The pre-defined time interval 310 may range from e.g. hours, several hours, or even a day.

Figure 6 depicts a scenario where the QR code 501 is scanned by a scanning device 601 such as any type of portable device, e.g. a mobile phone, and where the data embedded in the QR code is transmitted to the external control computer 209. The step of transmitting may as an example be tiggered by the scanning such that a service request is sent to the server individuated by the QR code and, using the same service request object's state data, the measurement data contained in the QR code is also transmitted the server.

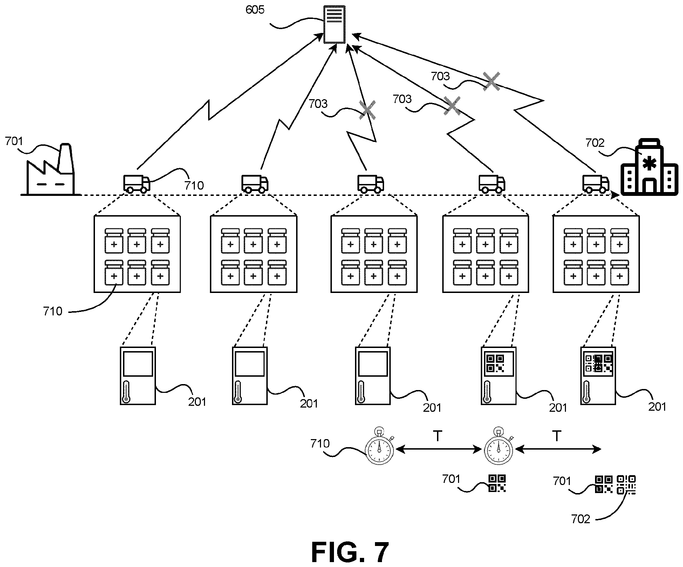

Figure 7 depicts a scenario where the asset 710 together with the logger device 201 is transported by a vehicle 610 from a begin location 701 to a destination location 702 during the real-time monitoring.

In this embodiment position data are further transmitted together with the stored measured environment related parameter to track the location of the vehicle in real-time and monitoring in real-time the storage conditions of the asset.

During the transport a communication gap 703 is detected, as discussed in relation to figure 6, which may be due to lack of cellular connection. From that time-point the processor converts the received and stored environment related parameters over a pre-defined time interval T, starting from the time point 710 where the communication gap is identified, into a Quick Response (QR) code and presents the QR code on the display. Moreover, the location data is embedded into the QR code since the location may also be a highly relative parameter.

In an embodiment, as indicated graphically here, when the resolution of the QR code 701 reaches the pre-defined resolution range a subsequent QR code 702 is generated and where the pre-defined time for the subsequent QR code is selected such that the resolution of the subsequent QR code when displayed on the display is above the pre-defined resolution range. This may be repeated multiple of times until the asset reaches the destination location 702, and the QR-codes are scanned as discussed in relation to figure 6.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

## Claims

1. A method of real-time monitoring of an asset such as a pharmaceutical product using a logger device associated with the asset, where the logger device comprises:
• a sensor for measuring an environment related parameter of the asset,
• a memory for storing the measured environment related parameter,
• a transmitter for transmitting via a communication network the stored measured environment related parameter to an external computer device with a pre-defined time interval,
• a display, and
• a processor for operating the sensor, the memory, the communication module and the display,
wherein the method comprises:
• identifying, by the processor, if there is a communication gap between the logger device and the external computer device, where in case a communication gap is identified,
• converting, by the processor, the received and stored environment related parameters over a pre-defined time interval starting from the time point where the communication gap is identified into a Quick Response (QR) code and present the QR code on the display.

2. The method according to claim 1, wherein the logger device is uniquely identified via a unique identification (ID), where the QR code further contains the unique ID.

3. The method according to claim 1 or 2, wherein the step of converting comprises:
• converting the received and stored environment related parameters over a discrete time interval into a QR code, where each discrete time interval is shorter than the pre-defined time interval, and
• gradually updating the QR code for each of said discrete time intervals with increased number of stored environment related parameters from previous time interval until the expiry of the pre-defined time interval.

4. The method according to any of the claims 1 or 2, wherein the step of converting comprises converting the received and stored environment related parameters accumulated over the pre-defined time interval into a single QR code.

5. The method according to any of the preceding claims, wherein the asset together with the logger device is stationary during the real-time monitoring and where the asset is placed in a temperature-controlled area.

6. The method according to any of the claims 1 to 4, wherein the asset together with the logger device is transported from a beginning location to a destination location during the real-time monitoring, where the method further comprises embedding position data to the QR code and transmitting the position data of the logger device together with the stored measured environment related parameter.

7. The method according to any of the preceding claims, further comprising compressing the received and stored environment related parameters prior to converting it into the QR code.

8. The method according to any of the preceding claims, wherein the pre-defined time interval is selected such that the resolution of the QR code when displayed on the display is above a pre-defined resolution range.

9. The method according to claim 8, wherein when the resolution of the QR code reaches the pre-defined resolution range a subsequent QR code is generated and where the pre-defined time interval for the subsequent QR code is selected such that the resolution of the subsequent QR code when displayed on the display is above the pre-defined resolution range.

10. The method according to claim 8, wherein when the resolution of the QR code reaches the pre-defined resolution range the resolution range is reduced.

11. The method according to any of the preceding claims, wherein the environmental related parameter is selected from one or more of:
• the temperature of the asset or around the asset,
• the ambience pressure,
• the vibration or acceleration of the asset,
• the light intensity around the asset.

12. The method according to any of the preceding claims, further comprising defining reference value or reference range of the environment related parameter indicating optimal transport conditions for the asset during the transport.

13. The method according to claim 12, wherein if an excursion occurs from the defined reference value or reference range the frequency of measured environment related parameter is increased.

14. The method according to any of the preceding claims, further comprising, upon an external action and prior to the expiry of the pre-defined time interval, a QR code is generated based on the stored data for a time-period starting from the time point where the communication gap is identified until the external action is received.

15. A system of real-time monitoring of an asset using a logger device associated with the asset while the asset, where the logger device comprises:
• a sensor for measuring an environment related parameter of the asset,
• a memory for storing the measured environment related parameter,
• a transmitter for transmitting via a communication network the stored measured environment related parameter to an external computer device with a pre-defined time interval,
• a display, and
• a processor for operating the sensor, the memory, the communication module and the display,
wherein the processor is configured to identify if there is a communication gap between the logger device and the external computer device, where in case a communication gap is identified, and convert the received and stored environment related parameters over a pre-defined time interval starting from the time point where the communication gap is identified into a Quick Response (QR) and present the QR code on the display.
